# EUROPEAN PATENT SPECIFICATION

(11) **EP 0 760 243 B1**
(45) Date of publication and mention of the grant of the patent: **29.03.2006**
(21) Application number: 95870100.5
(22) Date of filing: 31.08.1995
(51) Int. Cl.: A61L 9/01, C11D 3/50

(54) **Use of allylic alcohol perfumes as a malodour reduction agent**
Verwendung von Allylalkohol als ein Mittel zur Verminderung von schlechten Gerüchen
Utilisation de l'alcool allylique comme agent de réduction de mauvaises odeurs

(43) Date of publication of application: 05.03.1997
(73) Proprietor: THE PROCTER & GAMBLE COMPANY, Cincinnati, Ohio 45202 (US)
(72) Inventor: Groverman, Howard Victor, B-1410 Waterloo (BE); Rousset, Christophe Jacques, B-1853 Strombeek-Bever (BE)
(74) Representative: Engisch, Gautier

(56) References cited:
- EP-A- 0 142 886
- EP-A- 0 523 287
- WO-A-93/04158
- FR-A- 1 590 898
- FR-A- 2 666 510
- DATABASE WPI Section Ch, Week 9031 Derwent Publications Ltd., London, GB; Class D25, AN 90-236707 & JP-A-02 166 195 ( LION CORP) , 26 June 1990
- DATABASE WPI Section Ch, Week 8951 Derwent Publications Ltd., London, GB; Class A97, AN 89-372973 & JP-A-01 256 597 ( KAO CORP) , 13 October 1989

## Description

### Field of the invention

The present invention relates to the use of an allylic alcohol perfume precursor for reducing malodours selected from perspiration, smoke, kitchen smells and mixtures thereof.

### Background of the invention

Consumer acceptance of cleaning and laundry products is determined not only by the performance achieved with these products but the aesthetics associated therewith. Perfume systems are therefore an important aspect of the successful formulation of such commercial product. They are asserted to provide a fresh and clean character to the cleaned surface or laundered fabric.

WO-A-9504809 discloses a method which provides slow release of odoriferous alcohol, aldehyde or ketone via a hydrolisation of an ester compound. The said method is asserted to provide a clean and fresh character to the laundered fabrics.

EP-A-0,404,470 discloses perfumes fragrance which reduces malodours arising from perspiration.

WO-A-96 02625 discloses laundry and cleaning compositions containing nonionic or anionic ester of an allylic alcohol perfume for providing release of the perfume over a longer period of time than by the use of the perfume itself in the laundry/cleaning compositions.

Furthermore FR-A-26 66 510 discloses the use of geranyl acetate or neryl acetate for reducing maladours resulting from perspiration or smoke.

The Applicant has now found that another important aspect of successful formulation of commercial product is measured by the resulting malodour of the cleaned surface and/or laundered fabric; especially after exposure to strong malodours such as perspiration, smoke and/or kitchen odours. These malodours are among the most persistent odours on dried cleaned fabrics and/or surfaces.

The Applicant has now found that the use of allylic alcohol perfume precursors, wherein the alcohol precursor is selected from the group consisting of digeranyl succinate, dineryl succinate, geranyl neryl succinate, geranyl laurate, neryl laurate, and mixtures thereof overcomes the problem.

It is therefore an object of the invention to provide the use of said allylic alcohol perfume precursors thereof for reducing malodours arising from perspiration, smoke and/or kitchen odours.

### Summary of the invention

The present invention relates to the use of an allylic alcohol perfume precursor, wherein the allylic alcohol perfume precursor is selected from the group consisting of digeranyl succinate, dineryl succinate, geranyl neryl succinate, genaryl laurate, neryl laurate, and mixtures thereof, for reducing malodours selected from perspiration, smoke, kitchen smells and mixtures thereof.

In another preferred embodiment of the invention, the said precursor is incorporated in a cleaning or laundry composition.

### Detailed description of the invention

The present invention is directed to the use of allylic alcohol perfume precursor as defined in claim 1.

The preferred malodour is smoke such as cigarettes smoke and fire smoke on cleaned dried surfaces and/or laundered dried fabrics which are thereafter exposed to such malodours. Allylic alcohol perfume precursor

An essential component for the purpose of the invention is an allylic alcohol perfume precursor selected from the group consisting of digeranyl succinate, dineryl succinate, geranyl neryl succinate, geranyl laurate, neryl laurate, and mixtures thereof.

Geraniol and nerol are trans/cis structural isomers (at the 2,3 position double bond) of the molecules having the formula

HO-CH₂-CH=C(CH₃)-CH₂-CH₂-CH=C(CH₃)₂.

Used allylic alcohol perfume precursor esters have the following formulae : 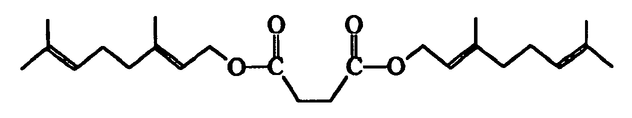 referred to herein as "digeranyl succinate" and 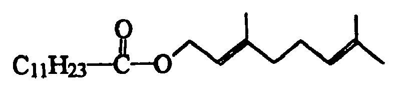 referred to herein as "geranyl laurate", as well as the neryl esters corresponding to these geranyl esters, including the mixed geranyl neryl succinate ester, and especially mixtures of the corresponding geranyl and neryl esters.

Most preferred esters for use herein are selected from digeranyl succinate, dineryl succinate, geranyl neryl succinate and mixtures thereof.

Also provided herein is the use according to the invention of said esters in laundry or cleaning products, which are typically used for laundering fabrics and cleaning hard surfaces such as dishware and other surfaces in need of cleaning and/or disinfecting.

Preferred are those laundry compositions which result in contacting the ester of the allylic alcohol perfume as described hereinbefore with fabric. These are to be understood to include not only detergent compositions which provide fabric cleaning benefits but also laundry compositions such as rinse added fabric softener compositions and dryer added compositions (e.g., sheets) which provide softening and/or antistatic benefits.
The allylic perfume ester(s) typically comprise from about 0.01% to about 10%, preferably from about 0.05% to about 5%, and more preferably from about 0.1% to about 2%, by weight of the composition.

Optional ingredients useful for formulating such laundry and cleaning compositions include one or more of the following.

### Fabric Softening Agents:

Suitable fabric softening agents for use herein are selected from cationic, nonionic fabric softening agents and mixtures thereof.

The preferred fabric softening agents to be used in the present compositions are quaternary ammonium compounds or amine precursors herein having the formula (I) or (II), below : 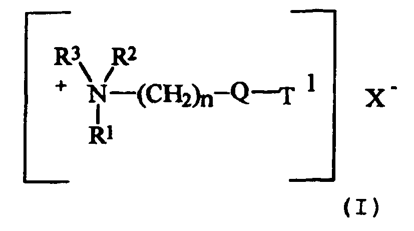 or 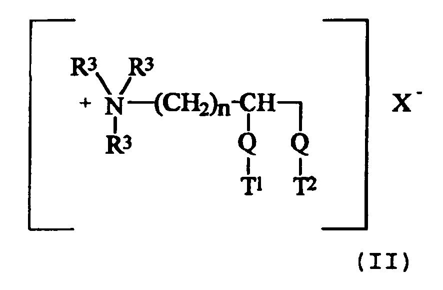 Q is selected from -O-C(O)-, -C(O)-O-, -O-C(O)-O-, -NR⁴⁻C(O)-, -C(O)-NR⁴-;
R¹ is (CH₂)ₙ-Q-T² or T³;
R² is (CH₂)ₘ-Q-T⁴ or T⁵ or R³;
R³ is C₁-C₄ alkyl or C₁-C₄ hydroxyalkyl or H;
R⁴ is H or C₁-C₄ alkyl or C₁-C₄ hydroxyalkyl;
T¹, T², T³, T⁴, T⁵ are (the same or different) C₁₁-C₂₂ alkyl or alkenyl;
n and m are integers from 1 to 4; and
X- is a softener-compatible anion, such as chloride, methyl sulphate, etc.

The alkyl, or alkenyl, chain T¹, T², T³, T⁴, T⁵ must contain at least 11 carbon atoms, preferably at least 16 carbon atoms. The chain may be straight or branched.

Tallow is a convenient and inexpensive source of long chain alkyl and alkenyl material. The compounds wherein T¹, T², T³, T⁴, T⁵ represents the mixture of long chain materials typical for tallow are particularly preferred.
Specific examples of quaternary ammonium compounds suitable for use in the aqueous fabric softening compositions herein include :
1) N,N-di(tallowyl-oxy-ethyl)-N,N-dimethyl ammonium chloride;
2) N,N-di(tallowyl-oxy-ethyl)-N-methyl, N-(2-hydroxyethyl) ammonium chloride;
3) N,N-di(2-tallowyloxy-2-oxo-ethyl)-N,N-dimethyl ammonium chloride;
4) N,N-di(2-tallowyloxyethylcarbonyloxyethyl)-N,N-dimethyl ammonium chloride;
5) N-(2-tallowoyloxy-2-ethyl)-N-(2-tallowyloxy-2-oxoethyl) -N,N-dimethyl ammonium chloride;
6) N,N,N-tri(tallowyl-oxy-ethyl)-N-methyl ammonium chloride;
7) N-(2-tallowyloxy-2-oxoethyl)-N-(tallowyl)-N,N-dimethyl ammonium chloride; and
8) 1,2-ditallowyloxy-3-trimethylammoniopropane chloride.;
and mixtures of any of the above materials.

Of these, compounds 1-7 are examples of compounds of Formula (I); compound 8 is a compound of Formula (II).

Particularly preferred is N,N-di(tallowoyl-oxy-ethyl)-N,N-dimethyl ammonium chloride, where the tallow chains are at least partially unsaturated.

The level of unsaturation of the tallow chain can be measured by the Iodine Value (IV) of the corresponding fatty acid, which in the present case should preferably be in the range of from 5 to 100 with two categories of compounds being distinguished, having a IV below or above 25.

Indeed, for compounds of Formula (I) made from tallow fatty acids having a IV of from 5 to 25, preferably 15 to 20, it has been found that a cis/trans isomer weight ratio greater than about 30/70, preferably greater than about 50/50 and more preferably greater than about 70/30 provides optimal concentrability.

For compounds of Formula (I) made from tallow fatty acids having a IV of above 25, the ratio of cis to trans isomers has been found to be less critical unless very high concentrations are needed.

Other examples of suitable quaternary ammoniums of Formula (I) and (II) are obtained by, e.g.,
- replacing "tallow" in the above compounds with, for example, coco, palm, lauryl, oleyl, ricinoleyl, stearyl, palmityl, or the like, said fatty acyl chains being either fully saturated, or preferably at least partly unsaturated;
- replacing "methyl" in the above compounds with ethyl, ethoxy, propyl, propoxy, isopropyl, butyl, isobutyl or t-butyl;
- replacing "chloride" in the above compounds with bromide, methylsulfate, formate, sulphate, nitrate, and the like.

In fact, the anion is merely present as a counterion of the positively charged quaternary ammonium compounds. The nature of the counterion is not critical at all to the practice of the present invention. The scope of this invention is not considered limited to any particular anion.

By "amine precursors thereof" is meant the secondary or tertiary amines corresponding to the above quaternary ammonium compounds, said amines being substantially protonated in the present compositions due to the claimed pH values.

The quaternary ammonium or amine precursors compounds herein are present at levels of from 1% to 80% of compositions herein, depending on the composition execution which can be dilute with a preferred level of active from 5% to 15%, or concentrated, with a preferred level of active from 15% to 50%, most preferably 15% to 35%.

For the preceding fabric softening agents, the pH of the compositions herein is an essential parameter. Indeed, it influences the stability of the quaternary ammonium or amine precursors compounds, especially in prolonged storage conditions.

The pH, as defined in the present context, is measured in the neat compositions at 20°C. For optimum hydrolytic stability of these compositions, the neat pH, measured in the above-mentioned conditions, must be in the range of from 2.0 to 4.5, preferably 2.0 to 3.5. The pH of these compositions herein can be regulated by the addition of a Bronsted acid.

Examples of suitable acids include the inorganic mineral acids, carboxylic acids, in particular the low molecular weight (C₁-C₅) carboxylic acids, and alkylsulfonic acids. Suitable inorganic acids include HCl, H₂SO₄, HNO₃ and H₃PO₄. Suitable organic acids include formic, acetic, citric, methylsulfonic and ethylsulfonic acid. Preferred acids are citric, hydrochloric, phosphoric, formic, methylsulfonic acid, and benzoic acids.

Softening agents also useful are nonionic fabric softener materials, preferably in combination with cationic softening agents.
Typically, such nonionic fabric softener materials have a HLB of from 2 to 9, more typically from 3 to 7. Such nonionic fabric softener materials tend to be readily dispersed either by themselves, or when combined with other materials such as single-long-chain alkyl cationic surfactant described in detail hereinafter. Dispersibility can be improved by using more single-long-chain alkyl cationic surfactant, mixture with other materials as set forth hereinafter, use of hotter water, and/or more agitation. In general, the materials selected should be relatively crystalline, higher melting, (e.g. >40°C) and relatively water-insoluble.

The level of optional nonionic softener in the compositions herein is typically from about 0.1% to about 10%, preferably from about 1% to about 5%.

Preferred nonionic softeners are fatty acid partial esters of polyhydric alcohols, or anhydrides thereof, wherein the alcohol, or anhydride, contains from 2 to 18, preferably from 2 to 8, carbon atoms, and each fatty acid moiety contains from 12 to 30, preferably from 16 to 20, carbon atoms. Typically, such softeners contain from one to 3, preferably 2 fatty acid groups per molecule.

The polyhydric alcohol portion of the ester can be ethylene glycol, glycerol, poly (e.g., di-, tri-, tetra, penta-, and/or hexa-) glycerol, xylitol, sucrose, erythritol, pentaerythritol, sorbitol or sorbitan. Sorbitan esters and polyglycerol monostearate are particularly preferred.

The fatty acid portion of the ester is normally derived from fatty acids having from 12 to 30, preferably from 16 to 20, carbon atoms, typical examples of said fatty acids being lauric acid, myristic acid, palmitic acid, stearic acid, oleic and behenic acid.

Highly preferred optional nonionic softening agents for use herein are the sorbitan esters, which are esterified dehydration products of sorbitol, and the glycerol esters.

Commercial sorbitan monostearate is a suitable material. Mixtures of sorbitan stearate and sorbitan palmitate having stearate/palmitate weight ratios varying between about 10:1 and about 1:10, and 1,5-sorbitan esters are also useful.

Glycerol and polyglycerol esters, especially glycerol, diglycerol, triglycerol, and polyglycerol mono- and/or diesters, preferably mono-, are preferred herein (e.g. polyglycerol monostearate with a trade name of Radiasurf 7248).

Useful glycerol and polyglycerol esters include monoesters with stearic, oleic, palmitic, lauric, isostearic, myristic, and/or behenic acids and the diesters of stearic, oleic, palmitic, lauric, isostearic, behenic, and/or myristic acids. It is understood that the typical monoester contains some di- and tri-ester, etc.

The "glycerol esters" also include the polyglycerol, e.g., diglycerol through octaglycerol esters. The polyglycerol polyols are formed by condensing glycerin or epichlorohydrin together to link the glycerol moieties via ether linkages. The mono- and/or diesters of the polyglycerol polyols are preferred, the fatty acyl groups typically being those described hereinbefore for the sorbitan and glycerol esters.

Additional fabric softening agents useful herein are described in U.S 4,661,269; U.S 4,439,335; U.S 3,861,870; 4, 308, 151; 3, 886, 075; 9, 233, 164; 4, 401, 578; 3,974,076; 4,237,016; and EP 472,178.

Fully formulated fabric softening compositions preferably contain, in addition to the hereinbefore described components, one or more of the following ingredients.

Concentrated compositions may require organic and/or inorganic concentration aids to go to even higher concentrations and/or to meet higher stability standards depending on the other ingredients. Surfactant concentration aids are typically selected from the group consisting of single long chain alkyl cationic surfactants; nonionic surfactants; amine oxides; fatty acids; or mixtures thereof, typically used at a level of from 0 to about 15% of the composition.

Inorganic viscosity control agents which can also act like or augment the effect of the surfactant concentration aids, include water-soluble, ionizable salts which can also optionally be incorporated into the compositions herein. A wide variety of ionizable salts can be used. Examples of suitable salts are the halides of the Group IA and IIA metals of the Periodic Table of the Elements, e.g., calcium chloride, magnesium chloride, sodium chloride, potassium bromide, and lithium chloride. The ionizable salts are particularly useful during the process of mixing the ingredients to make the compositions herein, and later to obtain the desired viscosity. The amount of ionizable salts used depends on the amount of active ingredients used in the compositions and can be adjusted according to the desires of the formulator. Typical levels of salts used to control the composition viscosity are from about 20 to about 20,000 parts per million (ppm), preferably from about 20 to about 11,000 ppm, by weight of the composition.

Alkylene polyammonium salts can be incorporated into the composition to give viscosity control in addition to or in place of the water-soluble, ionizable salts above. In addition, these agents can act as scavengers, forming ion pairs with anionic detergent carried over from the main wash, in the rinse, and on the fabrics, and may improve softness performance. These agents may stabilise the viscosity over a broader range of temperature, especially at low temperatures, compared to the inorganic electrolytes.

Specific examples of alkylene polyammonium salts include 1-lysine monohydrochloride and 1,5-diammonium 2-methyl pentane dihydrochloride.

Another optional, but preferred, ingredient is a liquid carrier. The liquid carrier employed in the instant compositions is preferably at least primarily water due to its low cost, relative availability, safety, and environmental compatibility. The level of water in the liquid carrier is preferably at least about 50%, most preferably at least about 60%, by weight of the carrier. Mixtures of water and low molecular weight, e.g., <about 200, organic solvent, e.g., lower alcohols such as ethanol, propanol, isopropanol or butanol are useful as the carrier liquid. Low molecular weight alcohols include monohydric, dihydric (glycol, etc.) trihydric (glycerol, etc.), and higher polyhydric (polyols) alcohols.

Still other optional ingredients are Soil Release Polymers, bacteriocides, colorants, perfumes, preservatives, optical brighteners, anti ionisation agents, antifoam agents, and the like.
Fully formulated cleaning or laundry compositions, such as detergent compositions preferably contain, in addition to the hereinbefore described components, one or more of the following ingredients selected from builders, bleaching agents with or without bleach activators, enzymes, enzymes stabilisers, detersive surfactants, chelating agents and mixtures thereof. Exemplary disclosure of such components may be found in WO 94/03572 and WO 94/03553.
Other preferred optional ingredients include polymeric soil release agents, materials effective for inhibiting the transfer of dyes from one fabric to another during the cleaning process (i.e., dye transfer inhibiting agents), polymeric dispersing agents, suds suppressors, optical brighteners or other brightening or whitening agents, chelating agents, fabric softening clay, anti-static agents, other active ingredients, carriers, hydrotropes, processing aids, dyes or pigments, solvents for liquid formulations, solid fillers for bar compositions, etc.

Liquid detergent compositions can contain water and other solvents as carriers. Low molecular weight primary or secondary alcohols exemplified by methanol, ethanol, propanol, and isopropanol are suitable. Monohydric alcohols are preferred for solubilizing surfactant, but polyols such as those containing from 2 to about 6 carbon atoms and from 2 to about 6 hydroxy groups (e.g., 1,3-propanediol, ethylene glycol, glycerine, and 1,2-propanediol) can also be used. The compositions may contain from 5% to 90%, typically 10% to 50% of such carriers.

Granular detergents can be prepared, for example, by spray-drying (final product density about 520 g/l) or agglomerating (final product density above about 600 g/l) the Base Granule. The remaining dry ingredients can then be admixed in granular or powder form with the Base Granule, for example in a rotary mixing drum, and the liquid ingredients (e.g., nonionic surfactant and perfume) can be sprayed on.

The detergent compositions herein will preferably be formulated such that, during use in aqueous cleaning operations, the wash water will have a pH of between about 6.5 and about 11, preferably between about 7.5 and 10.5. Laundry products are typically at pH 9-11. Techniques for controlling pH at recommended usage levels include the use of buffers, alkalis, acids, etc., and are well known to those skilled in the art.
The invention is illustrated in the following non limiting example, in which all percentages are on a weight basis unless otherwise stated.

### EXAMPLE

### Washing procedure

Two sets of fabric (i.e knitted cotton T-shirts) were treated with a liquid fabric softening composition in cold water.

The first set was treated with a fabric softening composition A while the second set was treated with a fabric softening composition B. The wash process employing composition A constitutes an embodiment of the invention while the wash process employing composition B is prior art.

The formulation are herein below defined:

| **Ingredient** | **A** | **B** |
|---|---|---|
| DEQA (1) | 19.2 | 19.2 |
| HCl | 0.02 | 0.02 |
| Silicone Antifoam (2) | 0.019 | 0.019 |
| Soil release polymer | 0.19 | 0.19 |
| CaCl₂ | 0.15 | 0.15 |
| Blue dye (3) | 0.005 | 0.005 |
| Perfume | 0.70 | 0.70 |
| Digeranyl Succinate (4) | 0.44 | - |
| Water | 79.276 | 79.716 |

| | | |
|---|---|---|
| (1) Di-(soft-tallowyloxyethyl) dimethyl ammonium chloride | | |
| (2) DC-110A silicon, sold by Dow-Corning | | |
| (3) Brilliant Polar Blue | | |
| (4) 1,4-Butandioic acid, 3,7-dimethyl-2,6-octadienyl ester | | |

### Malodour exposure procedure to smoke

The dried laundered fabrics were thereafter hanged for 1 minute in a 200 litres drum which had previously been saturated with cigarette smoke.
Saturation of the drum with cigarette smoke is obtained by placing a smoking cigarette in the closed drum for two minutes. Thereafter, the cigarette is removed to be replaced by the two set of fabric.
After exposure of the fabrics to cigarette smoke, the sets are removed from the drum and suspended in open air for 45 seconds, wrapped in aluminum foil during 24 hours and subsequently subject to sensory analysis.

### Sensory analysis

After smoke exposure, the perceived intensity of residual malodour on the fabric was carried out by a panel of 6 expert judges, trained to use sensory evaluations. In this instance, expert is defined as a person having at least 6 months training with demonstrated evidence of olfactive sensitivity. The data obtained using the perfumers intensity scale were averaged to give a consensus value for the perceived intensity of residual malodour.

The perfumers intensity scale is as follows:
0-no odour,
25- slight odour,
50- moderate odour,
75- very strong odour, and
100-extremely strong odour

### Results

| Treated T-shirts with | Malodour intensity after smoke exposure |
|---|---|
| Composition A | 25 |
| Composition B | 50 |

It is seen that the use of geraniol delivered by hydrolysis of digeranyl succinate (1,4-Butandioic acid, 3,7-dimethyl-2,6-octadienyl ester) in liquid fabric softening composition A provides an enhanced malodour reduction arising from smoke on dried cleaned fabric.

Identical results were obtained where fabric were exposed to kitchen smell according to the following test:
After being subject to the above mentionned washing procedure, the dried laundered fabrics (i.e knitted cotton T-shirts) were subject to the kitchen odour exposure according to the procedure below:
4 sets of fabric were worn by personal kitchen involved in cooking for 4 hours. Thereafter, the the sets are suspended in open air for 45 seconds, wrapped in aluminum foil during 24 hours and subsequently subject to the above mentionned sensory analysis.

Identical results were obtained when cotton garments were exposed to perspiration. After being subject to the above mentioned washing procedure the dried laundered garments were cut into two equal halves so that one half treated with product A could be put together with the complementary half treated with product B.
This "split item" was then worn by a panel of 20 persons during 8 hours (non-stressed) normal activity or 1-2 hours (stressed) sport session.
Thereafter the split items were evaluated on both halves according to the above mentioned sensory analysis.
Similar results were obtained when bed sheets, also cut in two equal halves, treated respectively with product A and B, were used by a panel of 20 users, on the basis of a 5-night exposure.

## Claims

1. Use of an allylic alcohol perfume precursor for reducing malodours selected from perspiration, smoke, kitchen smells and mixtures thereof, wherein the allylic alcohol perfume precursor is selected from the group consisting of digeranyl succinate, dineryl succinate, geranyl neryl succinate, geranyl laurate, neryl laurate, and mixtures thereof.

2. Use of an allylic alcohol perfume precursor according to claim 1, wherein said precursor is incorporated in a cleaning or laundry composition in amount of 0.05% to 5% by weight of the composition.

3. Use of an allylic alcohol perfume precursor according to claim 2, wherein said cleaning or laundry composition is a fabric softening composition comprising softening agents selected from the group consisting of cationic, nonionic fabric softening agents, and mixtures thereof.

4. Use of an allylic alcohol perfume precursor according to claim 3, wherein said fabric softening composition has a neat pH at 20°C in the range of from 2.0 to 4.5.

5. Use of an allylic alcohol perfume precursor according to claim 4, wherein said fabric softening composition has a neat pH at 20°C in the range of from 2.0 to 3.5.

## Revendications

1. Utilisation d'un précurseur parfum d'alcool allylique pour réduire les mauvaises odeurs choisies parmi la perspiration, la fumée, les odeurs de cuisine et leurs mélanges, précurseur parfum d'alcool allylique étant choisi parmi le groupe comprenant le succinate de digéranyle, le succinate de dinéryle, le succinate de géranyle néryle, le laurate de géranyle, le laurate de nétyle, et leurs mélanges.

2. Utilisation d'un précurseur parfum d'alcool allylique selon la revendication 1, ledit précurseur étant incorporé dans une composition de nettoyage ou de lavage du linge en une quantité de 0,05 % à 5 % en poids de la composition.

3. Utilisation d'un précurseur parfum d'alcool allylique selon la revendication 2, où ladite composition de nettoyage ou de lavage du linge est une composition d'adoucissement des tissus comprenant des agents adoucissants choisis dans le groupe comprenant des agents d'adoucissement des tissus cationiques, non ioniques, et leurs mélanges.

4. Utilisation d'un précurseur parfum d'alcool allylique selon la revendication 3, où ladite composition d'adoucissement des tissus a un pH pur à 20 °C dans la gamme allant de 2,0 à 4,5.

5. Utilisation d'un précurseur parfum d'alcool allylique selon la revendication 4, où ladite composition d'adoucissement des tissus a un pH pur à 20 °C dans la gamme allant de 2,0 à 3,5.

## Patentansprüche

1. Verwendung eines Allylalkohol-Duftstoffvorläufers zum Reduzieren schlechter Gerüche, ausgewählt aus Schweiß, Rauch, Küchengerüchen und Mischungen davon, worin der Allylalkohol-Duftstoffvorläufer ausgewählt ist aus der Gruppe, bestehend aus Digeranylsuccinat, Dinerylsuccinat, Geranylnerylsuccinat, Geranyllaurat, Neryllaurat und Mischungen davon.

2. Verwendung eines Allylalkohol-Duftstoffvorläufers nach nach Anspruch 1, worin der Vorläufer in einer Menge von 0,05 Gew.-% bis 5 Gew.-% der Zusammensetzung in eine Reinigungs- oder Wäschewaschzusammensetzung einbezogen wird.

3. Verwendung eines Allylalkohol-Duftstoffvorläufers nach Anspruch 2, worin die Reinigungs- oder Wäschewaschzusammensetzung eine Stoffweichmacherzusammensetzung ist, die Weichmacher, ausgewählt aus der Gruppe, bestehend aus kationischen, nichtionischen Stoffweichmachern und Mischungen davon, umfasst.

4. Verwendung eines Allylalkohol-Duftstoffvorläufers nach Anspruch 3, worin die Stoffweichmacherzusammensetzung bei 20 °C unverdünnt einen pH-Wert im Bereich von 2,0 bis 4,5 aufweist.

5. Verwendung eines Allylalkohol-Duftstoffvorläufers nach Anspruch 4, worin die Stoffweichmacherzusammensetzung bei 20 °C unverdünnt einen pH-Wert im Bereich von 2,0 bis 3,5 aufweist.
